# FASCICULE DE BREVET EUROPEEN

(11) **EP 2 379 501 B1**
(45) Date de publication et mention de la délivrance du brevet: **22.01.2014**
(21) Numéro de dépôt: 09802171.0
(22) Date de dépôt: 15.12.2009
(51) Int. Cl.: C07D 207/46, C07D 213/71, C07D 401/12

(54) **PROCEDE DE PREPARATION D'ESTERS ACTIVES**
VERFAHREN ZUR HERSTELLUNG AKTIVIERTER ESTER
METHOD FOR PREPARING ACTIVATED ESTERS

(30) Priorité: 17.12.2008 FR 0807090
(43) Date de publication de la demande: 26.10.2011
(73) Titulaire: SANOFI, 75008 Paris (FR)
(72) Inventeur: BIGOT, Antony, F-75013 Paris (FR)
(74) Mandataire: Gaslonde, Aude
(86) Numéro de dépôt international: PCT/FR2009/052528
(87) Numéro de publication internationale: WO 2010/076474

(56) Documents cités:
- WO-A-2004/016801
- TOKUTAKE NOBUYA ET AL: "Detection of unusual lipid mixing in cholesterol-rich phospholipid bilayers: the long and the short of it." JOURNAL OF THE AMERICAN CHEMICAL SOCIETY 30 JUL 2003, vol. 125, no. 30, 30 juillet 2003 (2003-07-30), pages 8994-8995, XP002528896 ISSN: 0002-7863
- CARLSSON J ET AL: "PROTEIN THIOLATION AND REVERSIBLE PROTEIN-PROTEIN CONJUGATION N-SUCCINIMIDYL 3-(2-PYRIDYLDITHIO)PROPIONATE, A NEW HETEROBIFUNCTIONAL REAGENT" BIOCHEMICAL JOURNAL, THE BIOCHEMICAL SOCIETY, LONDON, vol. 173, no. 3, 1 janvier 1978 (1978-01-01), pages 723-737, XP001146511 ISSN: 0264-6021
- OGURA ET AL: "A NOVEL ACTIVE ESTER SYNTHESIS REAGENT" TETRAHEDRON LETTERS, vol. 49, 1979, pages 4745-4746, XP002528897

## Description

La présente invention est relative à la préparation d'esters activés de formule (I) : 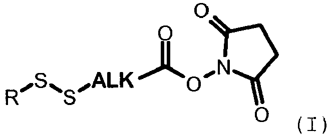 dans laquelle R représente un groupe (C₁-C₆)alkyle, aryle, hétéroaryle, cycloalkyle, hétérocycloalkyle et Alk représente un groupe (C₁-C₆)alkylène. Ces esters activés peuvent être utilisés dans la préparation de conjugués c'est-à-dire d'anticorps auxquels sont attachés par liaison covalente des composés chimiques biologiquement actifs tels que des composés cytotoxiques. On trouvera plus de détails sur la chimie des conjugués par exemple dans Birch and Lennox, Monoclonal Antibodies: Principles and Applications, Chap. 4, Wiley-Liss, New York, N.Y. (1995).

### [Art antérieur]

WO 2004/016801 décrit des esters activés comprenant un motif nitro-succinimide. Les préparations de ces composés décrites sur les figures 1 à 6 reposent sur des réactions différentes de celle envisagée dans la présente invention.

J. Med. Chem. 2006, 49(14), 4392-4408 décrit la préparation d'esters activés, notamment le N-succinimidyl-[4-méthyl-4-(méthyldithio)]pentanoate sur le schéma 6, par des réactions différentes de celle envisagée dans la présente invention.

Langmuir 2000, 16(1), 81-86 décrit sur le schéma 1 la préparation du succinimidyl-3-(2-pyridyldithio)butyrate (SPDB) par couplage de l'acide correspondant avec la N-hydroxysuccinimide.

US 6407263**,** US 5872261**,** US 5892057 et US 5942628 décrivent des esters activés et leur méthode de préparation.

Can. J. Chem. 1982, 60, 976 décrit la préparation du sel de dicyclohexylamine de la N-hydroxysuccinimide (P₂) par réaction entre la dicyclohexylamine et de la N-hydroxysuccinimide dans l'acétone. Ce composé a pour N° CAS 82911-72-6.

Can. J. Chem. 1986, 64(11), 2097-2102 ; J. Chem. Soc., Perkin Trans. 1 1985, 4, 765-8 ; Bull. Soc. Chem. Jpn 1986, 59(8), 2505-8 ; Coll. Czech. Chem. Comm. 1985, 50(12), 2925-2936 décrivent la préparation d'esters succinimide à partir de sels de dicyclohexylamine mais sans utiliser de carbonate de disuccinimidyle.

Tetrahedron Letters 1979, 49, 4745-4746 décrit le DSC et son intérêt en synthèse (voir schéma 2).

Biochem. J. 1978, 173, 723-737 décrit la prépration d'esters activés en présence de N-hydroxysuccinimide et de dicyclohexylcarbodiimide.

JACS 2003, 125(30), 8994-8995 relève de l'arrière plan technologique.

### [Brève description de l'invention]

L'invention est relative à un procédé de préparation d'un ester activé de formule (I) : 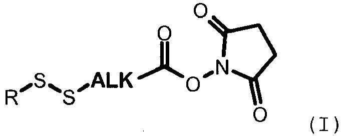 dans laquelle R représente un groupe (C₁-C₆)alkyle, linéaire ou ramifié, aryle, hétéroaryle, cycloalkyle, hétérocycloalkyle et Alk représente un groupe (C₁-C₆)alkylène, linéaire ou ramifié, consistant à faire réagir le sel de dicyclohexylamine P₁ : 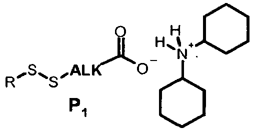 et le carbonate de disuccinimidyle (DSC) dans un solvant dans lequel le sel dicyclohexylamine de la N-hydroxysuccinimide P₂ 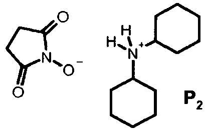 précipite.

L'invention est aussi relative aux produits de formule P₁ : 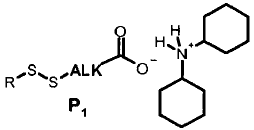 plus particulièrement ceux de formule :

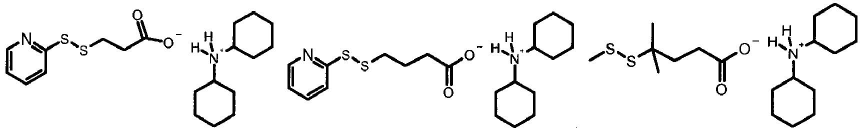

### [Description de l'invention]

### définitions

- groupe alkyle : un groupe hydrocarboné aliphatique saturé, linéaire ou ramifié, obtenu en enlevant un atome d'hydrogène d'un alcane. On peut notamment citer les groupes suivants : méthyle, éthyle, propyle, butyle, pentyle, hexyle, 2-méthylbutyle, 2-méthylpentyle, 1-méthylpentyle ;
- groupe alkylène : un groupe divalent obtenu en enlevant deux atomes d'hydrogène d'un alcane. On peut notamment citer les groupes suivants : méthylène (-CH₂-), éthylène (-CH₂CH₂-), n-propylène (-CH₂CH₂CH₂-), butylène (-CH₂CH₂CH₂CH₂-) ;
- groupe cycloalkyle : un groupe alkyle acyclique ayant de 3 à 10 atomes de carbone engagés dans la structure cyclique. On peut citer notamment les groupes suivants : cyclopropyle, cyclopentyle, cyclohexyle ;
- groupe aryle : un groupe aromatique de 6 à 10 atomes de carbone. On peut citer notamment les groupes suivants : phényle, napthyle, indényle, fluorényle ;
- groupe hétéroaryle : un groupe aromatique de 5 à 10 chaînons comprenant comme atomes formant le cycle, un ou plusieurs hétéroatomes choisis parmi O, S ou N ;
- groupe hétérocycloalkyle : un groupe cycloalkyle tel que défini précédemment comprenant en outre comme atome(s) formant le cycle, un ou plusieurs hétéroatomes choisis parmi N, O ou S.

La préparation repose sur la réaction entre le sel de dicyclohexylamine P₁ et le carbonate de disuccinimidyle (DSC) dans un solvant dans lequel le sel de dicyclohexylamine de la N-hydroxysuccinimide P₂ précipite **(Schéma I).**

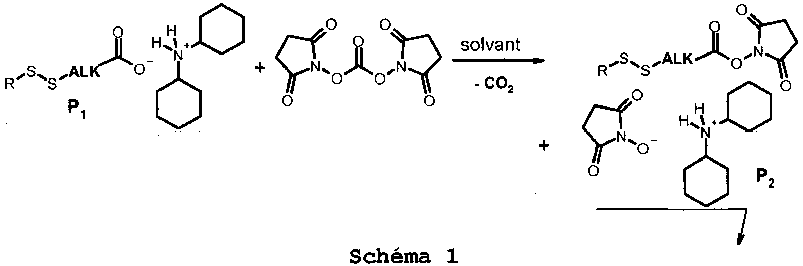

R représente un groupe :
- (C₁-C₆)alkyle : par exemple un groupe méthyle, éthyle, propyle, butyle ou pentyle, éventuellement ramifié ;
- (C₃-C₇)cycloalkyle : par exemple le groupe cyclopropyle ;
- aryle : par exemple le groupe phényle ;
- hétéroaryle : par exemple le groupe 2-pyridinyle ( 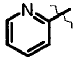 );
- hétérocycloalkyle : par exemple le groupe pipéridinyle.

Alk représente un groupe (C₁-C₆)alkylène, par exemple un groupe propylène, butylène ou pentylène, éventuellement ramifié. Il s'agit plus particulièrement du groupe (CH₂)ₙ, n désignant un nombre entier allant de 1 à 6.

La fonction de la dicyclohexylamine est de promouvoir la réaction et d'insolubiliser la N-hydroxyl-succinimide qui est libérée. Cette réaction présente les avantages suivants :
- facilité de mise en oeuvre : simple mise en contact, pas de chauffage, libération lente et contrôlée de CO₂ ;
- le composé P₁ étant sous forme de carboxylate, il n'est pas nécessaire d'ajouter une base supplémentaire pour activer la réaction ;
- le composé P₂ qui est libéré n'est que très peu soluble dans le solvant employé et il précipite. La majeure partie de P₂ peut donc facilement être éliminée par une simple séparation mécanique, une filtration par exemple ;
- la réaction permet d'obtenir facilement l'ester activé avec un bon rendement et une bonne pureté.

P₂ est préparé par neutralisation de l'acide correspondant par la dicyclohexylamine. Le DSC est un produit commercial.

Le solvant est une cétone qui présente moins de problèmes toxicologiques que les solvants habituellement utilisés pour ce type de réaction (dichlorométhane ou diméthylformamide). La cétone peut être par exemple l'acétone ou la méthyl-isobutyl-cétone (MIBK). La MIBK est préférée car étant peu miscible à l'eau (1,55% p/p à 20°C), elle permet un lavage aqueux du produit, facilitant ainsi l'élimination de P₂ résiduel. Elle permet également d'éliminer l'eau résiduelle par distillation azéotropique. Enfin, la MIBK est un bon solvant de l'ester activé mais pas des composés P₂, P₁ et de le DSC, ce qui permet une réaction lente et ménagée entre P₁ et le DSC : les réactifs peuvent être ainsi mélangés initialement en totalité sans que cela ne pose de problème en termes de sécurité (réaction rapide avec libération incontrôlée de CO₂).

La réaction est conduite à température ambiante (environ 20°C). P₂ peut précipiter spontanément dans certains solvants. Afin de favoriser la précipitation de P₂, on peut, après avoir fait réagir P₁ et le DSC, refroidir le mélange réactionnel (par exemple à une température proche de 0°C).

Cette réaction permet notamment de préparer les esters activés suivants : le N-succinimidyl-3-(2-pyridyldithio)-propionate (SPDP), le N-succinimidyl-3-(2-pyridyldithio)-butyrate (SPDB) ou le N-succinimidyl-[4-méthyl-4-(méthyldithio)]pentanoate à partir des sels d'acide correspondants à savoir respectivement :

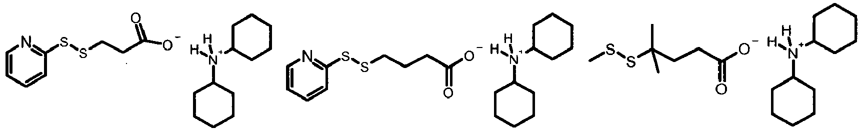

### [Exemples]

### Exemple 1 : préparation du N-succinimidyl-[4-méthyl-4-(méthyldithio)]pentanoate

La réaction est la suivante :

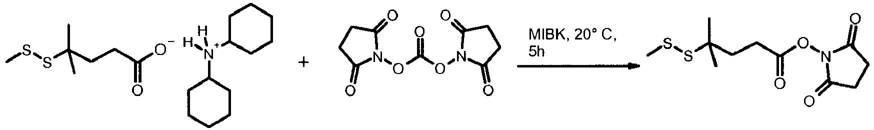

Une suspension du sel de dicyclohexylamine de l'acide 4-méthyl-4-(méthyldithio)pentanoïque (23 g) et de DSC (18,2 g, 1,1 éq.) dans 161 ml de MIBK est agitée à environ 20°C pendant 5 h. La suspension est alors refroidie à environ 0°C, agitée 1 h à cette température, puis filtrée.

Le solide est lavé par 2x23 ml de MIBK. Les phases organiques sont réunies, lavées par 2x58 ml d'une solution aqueuse d'HCl 6N, puis par 92 ml d'eau déminéralisée. La phase organique est alors concentrée à sec sous vide. Le solide résultant est solubilisé dans 230 ml de dichlorométhane (DCM) et la solution résultante est traitée par 46 g de silice, agitée 10 min, puis la silice est filtrée et lavée par 2x69 ml de DCM. Cette opération est répétée une seconde fois. La phase organique est alors concentrée à environ 2 volumes, puis, à environ 20°C, 391 ml de n-heptane sont ajoutés en environ 30 min. La suspension blanche résultante est agitée à cette température pendant environ 1 h, refroidie à environ -10°C en environ 1 h, puis agitée à cette température pendant environ 1 h. Le solide est alors filtré, lavé par 2x23 ml de n-heptane refroidi à environ -10°C, puis séché sous vide à 40°C pendant 15 h. Le 4-N-hydroxysuccinimidyl-[4-méthyl-4-(méthyldithio)]pentanoate est isolé avec un rendement de 70,6%. Sa pureté déterminée par HPLC est de 99,65 % (hors solvants).

### Exemple 2 : préparation du N-succinimidyl-3-(2-pyridyldithio)-butyrate (SPDB)

La réaction est la suivante :

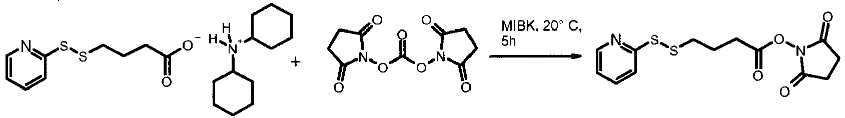

Le sel de dicyclohexylamine (40 g, 1 éq.) et le DSC (28,7 g, 1,1 éq.) sont mis en suspension dans 280 ml de MIBK. Le mélange est agité pendant 4 h à 20±3°C. La suspension est refroidie à 0±3°C, laissée 30 min à cette température, filtrée et le solide obtenu est lavée par de la MIBK glacée (120 ml). Les jus mères sont lavés avec de l'eau (3x176 ml) et évaporés à sec sous pression réduite au rotavapor avec un bain à 50°C jusqu'à obtenir une quantité de MIBK≤2,5 %. Le SPDB brut est obtenu sous forme d'huile jaune.

Le SPDB (32,5 g) est ensuite dissout dans de l'éthanol (455 ml) à 35±2°C. La solution obtenue est refroidie à 18±2°C : le SPDB pur commence à cristalliser. 90 ml de n-heptane sont ajoutés en 10 min, la cristallisation s'intensifie. Le mélange est refroidi à 0±3°C et 820 ml de n-heptane sont ajoutés en 20 min. Le mélange est agité pendant 1 h à 0±3°C. Le SPDB pur est isolé par filtration, lavé avec 2x90 ml de n-neptane glacé et séché à l'étuve (30°C, 50 mbar). rendement : 84,8%, pureté HPLC : 98,7%.

## Revendications

1. Procédé de préparation d'un ester activé de formule (I) 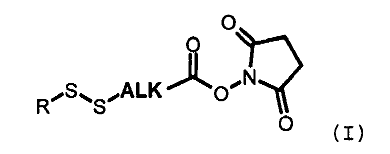 dans laquelle R représente un groupe (C₁-C₆)alkyle, linéaire ou ramifié, aryle, hétéroaryle, cycloalkyle, hétérocycloalkyle et Alk représente un groupe (C₁-C₆)alkylène, linéaire ou ramifié,
consistant à faire réagir le sel de dicyclohexylamine P₁ 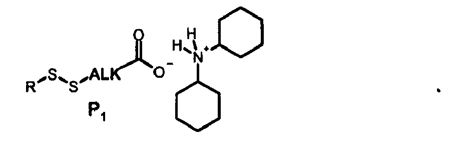 et le carbonate de disuccinimidyle (DSC) dans un solvant dans lequel le sel dicyclohexylamine de la N-hydroxysuccinimide P₂ 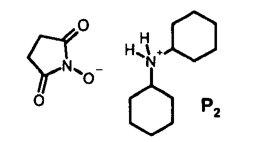 précipite, dans lequel ledit solvant est une cétone.

2. Procédé selon la revendication 1 dans lequel R représente un groupe méthyle, éthyle, propyle, butyle ou pentyle, éventuellement ramifié ou le groupe 2-pyridinyle.

3. Procédé selon la revendication 1 ou 2 dans lequel Alk représente un groupe propylène, butylène ou pentylène, éventuellement ramifié.

4. Procédé selon la revendication 1 ou 2 dans lequel Alk représente le groupe (CH₂)ₙ, n désignant un nombre entier allant de 1 à 6.

5. Procédé selon la revendication 1 dans lequel la cétone est la MIBK.

6. Procédé selon l'une quelconque des revendications 1 à 5 dans lequel après avoir fait réagir P₁ et le DSC, on refroidit le mélange réactionnel pour favoriser la précipitation de P₂.

7. Procédé selon l'une des revendications 1 à 6 dans lequel P₂ est éliminé par une séparation mécanique.

8. Procédé selon la revendication 7 dans lequel la séparation mécanique est une filtration.

9. Produit de formule P₁ : 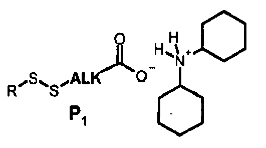 dans laquelle R et Alk sont tels que définis à l'une des revendications 1 à 4.

10. Produit selon la revendication 9 de formule :

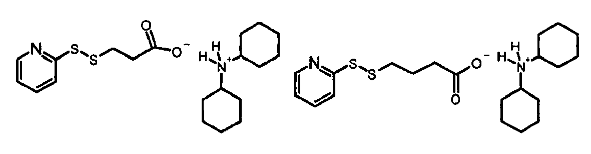

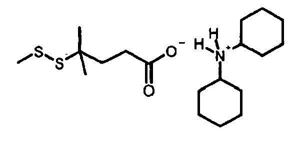

## Patentansprüche

1. Verfahren zur Herstellung eines aktivierten Esters der Formel (I) 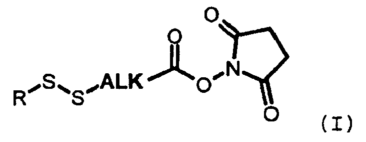 in der R für eine lineare oder verzweigte (C₁-C₆)-Alkyl-, Aryl-, Heteroaryl-, Cycloalkyl- oder Heterocycloalkylgruppe steht und Alk für eine lineare oder verzweigte (C₁-C₆)-Alkylengruppe steht,
bei dem man das Dicyclohexylaminsalz P₁ 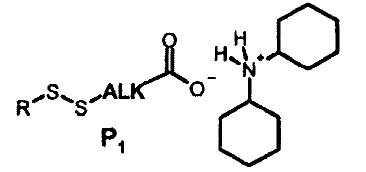 und Disuccinimidylcarbonat (DSC) in einem Lösungsmittel, in dem das Dicyclohexylaminsalz von N-Hydroxysuccinimid P₂ 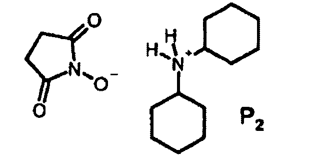 ausfällt, umsetzt, wobei es sich bei dem Lösungsmittel um ein Keton handelt.

2. Verfahren nach Anspruch 1, bei dem R für eine Methyl-, Ethyl-, Propyl-, Butyl- oder Pentylgruppe, die gegebenenfalls verzweigt ist, oder die 2-Pyridinylgruppe steht.

3. Verfahren nach Anspruch 1 oder 2, bei dem Alk für eine Propylen-, Butylen- oder Pentylengruppe, die gegebenenfalls verzweigt ist, steht.

4. Verfahren nach Anspruch 1 oder 2, bei dem Alk für die Gruppe (CH₂)ₙ steht, wobei n eine ganze Zahl im Bereich von 1 bis 6 bedeutet.

5. Verfahren nach Anspruch 1, bei dem es sich bei dem Keton um MIBK handelt.

6. Verfahren nach einem der Ansprüche 1 bis 5, bei dem man nach der Umsetzung von P₁ und DSC die Reaktionsmischung zur Förderung der Ausfällung von P₂ abkühlt.

7. Verfahren nach einem der Ansprüche 1 bis 6, bei dem man P₂ durch eine mechanische Trennung entfernt.

8. Verfahren nach Anspruch 7, bei dem es sich bei der mechanischen Trennung um eine Filtration handelt.

9. Produkt der Formel P₁: 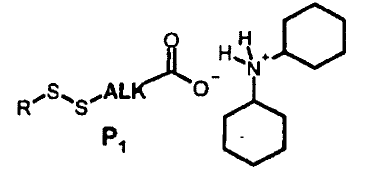 in der R und Alk wie in einem der Ansprüche 1 bis 4 definiert sind.

10. Produkt nach Anspruch 9 der Formel:

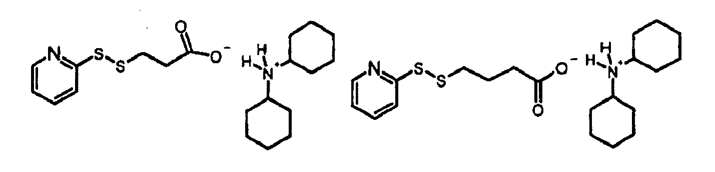

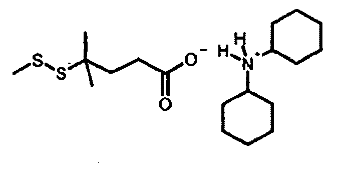

## Claims

1. Method for preparing an activated ester of formula (I) 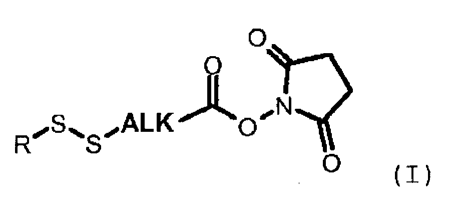 in which R is a linear or branched (C₁-C₆)alkyl, aryl, heteroaryl, cycloalkyl or heterocycloalkyl group, and Alk is a linear or branched (C₁-C₆)alkylene group,
said method consisting in reacting the dicyclohexylamine salt P₁: 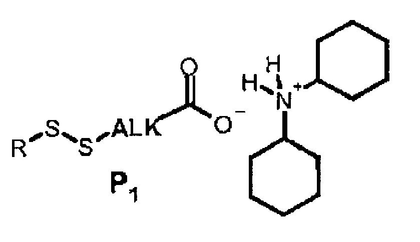 and disuccinimidyl carbonate (DSC) in a solvent in which the dicyclohexylamine salt of N-hydroxysuccinimide P₂ 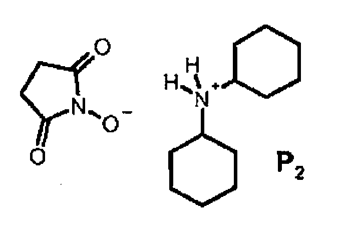 precipitates, said solvent being a ketone.

2. Method according to Claim 1, in which R is a methyl, ethyl, propyl, butyl or pentyl group, which is optionally branched, or the 2-pyridinyl group.

3. Method according to Claim 1 or 2, in which Alk is a propylene, butylene or pentylene group, which is optionally branched.

4. Method according to Claim 1 or 2, in which Alk is the (CH₂)ₙ group, n denoting an integer ranging from 1 to 6.

5. Method according to Claim 1, in which the ketone is MIBK.

6. Method according to any one of Claims 1 to 5, in which, after having reacted P₁ and DSC, the reaction mixture is cooled in order to promote the precipitation of P₂.

7. Method according to one of Claims 1 to 6, in which P₂ is removed by mechanical separation.

8. Method according to Claim 7, in which the mechanical separation is filtration.

9. Product of formula P₁: 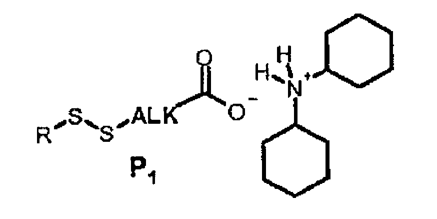 in which R and Alk are as defined in one of Claims 1 to 4.

10. Product according to Claim 9, of formula:
